## Europäisches Patentamt

## European Patent Office

## Office européen des brevets

(19)

(11) Veröffentlichungsnummer: **0 137 221**
**B1**

(12)

# EUROPÄISCHE PATENTSCHRIFT

(45) Veröffentlichungstag der Patentschrift :
**19.07.89**

(51) Int. Cl.⁴ : **B 01 D 15/08**, A 61 K 35/16

(21) Anmeldenummer : **84109651.4**

(22) Anmeldetag : **14.08.84**

(54) Verfahren zum Abtrennen von Lipoproteinen mittels derivatisiertem Polyhydroxymethylen.

(30) Priorität : 25.08.83 DE 3330648

(43) Veröffentlichungstag der Anmeldung :
**17.04.85 Patentblatt 85/16**

(45) Bekanntmachung des Hinweises auf die Patenterteilung : **19.07.89 Patentblatt 89/29**

(84) Benannte Vertragsstaaten :
**AT BE CH DE FR GB IT LI LU NL SE**

(56) Entgegenhaltungen :
CH--A-- 495 763
DE--A-- 2 404 041
DE--A-- 2 556 759
DE--A-- 2 751 528
DE--B-- 2 421 789
FR--A-- 2 343 251

(73) Patentinhaber : BEHRINGWERKE Aktiengesellschaft
Postfach 1140
D-3550 Marburg 1 (DE)

(72) Erfinder : Schmidtberger, Rudolf
Salegrund 1
D-3550 Marburg (DE)

(74) Vertreter : Meyer-Dulheuer, Karl-Hermann, Dr. et al
HOECHST Aktiengesellschaft Zentrale Patentabteilung Postfach 80 03 20
D-6230 Frankfurt/Main 80 (DE)

## Beschreibung

Die Erfindung betrifft ein Verfahren zum Abtrennen von Lipoproteinen aus wässrigen Flüssigkeiten durch Binden an Polyhydroxymethylen, das einen aufgepfropften oxethylierten Alkohol oder eine aufgepfropfte oxethylierte Carbonsäure enthält.

Bei der Bereitstellung haltbarer Plasmen und Seren aus menschlichem oder tierischem Blut für therapeutische oder diagnostische Zwecke hat, es sich als zweckmäßig erwiesen, alle labilen Proteine, soweit sie nicht für die vorgesehene Anwendung erforderlich sind, zu entfernen. Zu diesen, die Haltbarkeit der oben genannten Lösungen besonders beeinträchtigenden Plasma- und Serumbestandteilen Zählen die Lipoproteine.

Die Lipoproteine sind die wasserlösliche Transportform für Triglyceride, Cholesterinester und Phospholipide in biologischen Flüssigkeiten. Ihre Wasserlöslichkeit verdanken sie Anteilen von Protein, welche die an sich wasserunlöslichen Lipide hüllenartig umgeben. Im Extremfall kann der Proteinanteil am Lipoprotein nur wenige Prozent betragen. Der mehr oder weniger große Anteil an wasserunlöslichen Lipiden ist auch die Ursache für deren im Vergleich zu Proteinen und Glykoproteinen geringe Lösungsstabilität. Diese Lösungsstabilität ist jedoch sowohl bei therapeutisch als auch bei diagnostisch anzuwendenden biologischen Flüssigkeiten, wie beispielsweise Plasmen oder Seren, eine unumgängliche Voraussetzung für deren Anwendung. Es hat daher nicht an Bemühungen gefehlt, die Lipoproteine aus Plasmen oder Seren zu entfernen, ohne dabei die für den Anwender wichtigen biologischen Aktivitäten zu verändern. Diese Veränderung kann beispielsweise in einer Verminderung des Antikörpergehaltes eines Antiserums oder in der unerwünschten Aktivierung eines Gerinnungsfaktors bestehen.

Nach dem Stand der Technik stehen drei Methoden zur Entfernung der Lipoproteine aus biologischen Flüssigkeiten wie Plasma oder Serum zur Verfügung :

Die Methode der Flotation der Lipoproteine bei erhöhter Dichte erfordert den Einsatz hochtouriger Zentrifugen. Die Entfernung sämtlicher Lipoproteine aus Serum erfordert eine Erhöhung der Dichte auf 1,21, was in der Regel durch Zugabe von Kaliumbromid geschieht. Die Notwendigkeit der Rückführung der Nichtlipoproteine in ein physiologisches Milieu vor deren therapeutischer Anwendung stellt ein weiteres Hindernis für die Übertragung dieser Technik auf große Volumina dar.

Zur Adsorption der Lipoproteine an selektiv bindende Adsorbentien wurden eine Reihe von Adsorbentien beschrieben, deren gemeinsames Merkmal eine perlförmige hydrophile Matrix auf Polysaccharidbasis ist, an die Phenyl- oder Alkylgruppen gebunden sind. Nachteilig sind unerwünschte Wechselwirkungen mit Komponenten der biologischen Lösungen wie Fibrinogen aber auch ihre enzymatische Angreifbarkeit. Zu den Lipoproteine-bindenden Adsorbentien gehören auch solche auf der Basis von Siliziumdioxid. Diese unter der Bezeichnung Aerosil$^R$ im Handel befindlichen Siliziumdioxide können nur im Rührverfahren angewendet werden, wobei jedoch vom Sediment Flüssigkeit mit gelösten Stoffen eingeschlossen wird. Diese können zwar durch einen Waschvorgang wiedergewonnen werden, liegen dann aber in nicht verwendbarer verdünnter Form vor. Ein weiterer Nachteil der Siliziumdioxid-Adsorbention liegt in ihrer merklichen Löslichkeit bei pH-Werten von 7 und darüber. Auch die Selektivität ist in vielen Fällen unzureichend, so daß es beispielsweise nicht möglich ist, menschliches oder tierisches Plasma unter Erhaltung ihrer Gerinnungseigenschaften von den Lipoproteinen zu befreien.

Weiterhin ist bekannt, daß Lipoproteine mit Polyanionen wie Dextransulfat oder Heparin ausgefällt werden können. Dabei müssen Kationen wie $Mg^{++}$ oder $Mn^{++}$ anwesend sein. Die im Überstand verbliebenen Mengen an Polyanionen und Metallionen müssen dann entfernt werden.

Deshalb war es nicht möglich, Plasmen oder Seren menschlichen oder tierischen Ursprungs im 100 1-Maßstab von den Lipoproteinen zu befreien, wenn eine Veränderung der Elektrolytverhältnisse und des pH-Wertes, eine Verdünnung des Plasmas oder Serums, die Gefahr der Einschleppung von zu Unverträglichkeiten bei der therapeutischen Anwendung führenden Substanzen wie bakteriellen Pyrogenen und eine Änderung der Mengenverhältnisse der in der Lösung verbleibenden Proteine vermieden werden sollen. Außerdem soll das Verfahren unter sterilen Bedingungen anwendbar sein.

Aufgabe der Erfindung war es deshalb, ein Verfahren zur Entfernung von Lipoproteinen aus wässrigen Flüssigkeiten zu finden, das diesen Bedingungen genügt.

Überraschenderweise wurde gefunden, daß wasserunlösliches Polyhydroxymethylen, auf das ein oxethylierter Alkohol oder eine oxyethylierte Carbonsäure, insbesondere aliphatische Carbonsäure, aufgepfropft wurde, Lipoproteine aus wässrigen Flüssigkeiten zu binden vermag.

Gegenstand der Erfindung ist daher ein Verfahren zum Abtrennen von Lipoproteinen aus einer wässrigen Flüssigkeit, dadurch gekennzeichnet, daß die Flüssigkeit mit einem Polyhydroxymethylen in Kontakt gebracht wird, das einen aufgepfropften oxethylierten Alkohol oder eine aufgepfropfte oxethylierte Carbonsäure enthält. Bevorzugt werden als Alkohol ein Alkanol mit 4 bis 30 Kohlenstoffatomen oder eine aliphatische Carbonsäure mit 4 bis 20 Kohlenstoffatomen.

Ein solches derivatisiertes Polyhydroxymethylen (PHM), kann nach DE-OS 25 56 759 (USP 4,098,771) hergestellt werden.

Besonders geeignet ist ein PHM, das nach den Beispielen dieser Patentschrift hergestellt wurde.

In der Polymerisation werden von 1 bis 20, vorzugsweise 2 bis 10 Gewichtsprozent eines oxethylierten Alkohols oder einer oxethylierten Carbonsäure eingesetzt.

Die besonderen Vorteile eines derartigen Polymeren liegen in einer Reihe von Stoffeigenschaften, durch die es sich von anderen bekannten Adsorbentien unterscheidet. Polyhydroxymethylen ist ein synthetisches Polymer mit einer durchgehenden Kohlenstoffkette, in das in einer bevorzugten Herstellungsform über Ätherbindungen Alkylgruppen eingebaut werden. Das Endprodukt enthält also nur Bindungsarten, die sowohl für eine hohe chemische und thermische als auch für eine gute enzymatische Stabilität bekannt sind.

Durch das Fehlen von ionogenen Gruppen ist Polyhydroxymethylen zu keinen unerwünschten ionogen Wechselwirkungen fähig.

Die mit jedem der C-Atome des Polyhydroxymethylen verknüpften OH-Gruppen sorgen für eine gute Benetzbarkeit des Adsorbens mit wässrigen Lösungen, was sich vorteilhaft auf die Erhaltung der nativen Struktur von Proteinen auswirkt, deren Lösungen damit behandelt werden. Mit oxethyliertem Alkohol derivatisiertes Polyhydroxymethylen kann unter Ausnutzung seiner Alkalistabilität mit heißer Alkalilauge behandelt werden, nach deren Neutralisation sich eine sterile und pyrogenfreie Anwendung anschließen kann. Die Möglichkeit einer derartigen Maßnahme ist im Zusammenhang mit der Aufarbeitung therapeutisch anzuwendender Proteinlösungen von großer Bedeutung. Obwohl ein im Sinne des Anspruches derivatisiertes Polyhydroxymethylen sowohl innerhalb eines weiten pH-Bereiches als auch aus Lösungen unterschiedlicher Elektrolytart und -konzentration heraus Lipoproteine binden kann, ist es von besonderem Vorteil, daß es diese Eigenschaft bei der Anwendung auf Plasmen und Seren auch bei physiologischen Salz- und pH-Verhältnissen zeigt. Dadurch erübrigt sich eine vorherige Veränderung der Reaktionsbedingungen und deren Rückgängigmachung nach dem Adsorptionsschritt.

Die Bindung der Lipoproteine kann im Rührverfahren oder in der Säulentechnik erfolgen.

Das Polyhydroxymethylen kann in hydratisierter oder lyophilisierter Form angewendet werden. Im letzteren Fall ist die Möglichkeit gegeben, durch Pressen dem Adsorbens eine bestimmte Form zu geben. Zweckmäßigerweise wird das Polyvinylencarbonat, aus dem das PHM mittels Hydrolyse hergestellt wird, durch Polymerisation in Gegenwart von Dispergatoren gemäß EP-A-0 110 281 (Priorität: 25.11.82; Veröffentlichungstag: 13-06-84) gewonnen. Dadurch, daß das derivatisierte Polyhydroxymethylen aus einer Alkalilösung gefällt wird, ist die Möglichkeit gegeben, die Fällung in Anwesenheit feinverteilter Partikel vorzunehmen. Diese werden von dem ausfallenden Polyhydroxymethylen eingeschlossen und können so dem Copräzipität Eigenschaften verleihen, die die nachfolgende Verwendung erleichtern oder überhaupt erst ermöglichen. So kann der Einschluß von Magnetitpartikeln eine magnetische Beeinflußbarkeit verleihen. Durch den Einschluß von Kieselgur ist es beispielsweise möglich, das Fließverhalten von Polyhydroxymethylen-Säulenfüllungen zu verbessern. Eingeschlossene Kohlepartikel können zusätzliche Adsorptionseffekte bringen.

Sollen die an das Polyhydroxymethylen gebundenen Lipoproteine gewonnen werden, so stehen dafür alle Mittel der Elution zur Wahl, die für die Dissoziation hydrophober Bindungen bekannt sind.

Das folgende Beispiel erläutert die Erfindung.

Beispiel

200 g Polyvinylencarbonat, hergestellt nach USP 4098771, wurden in einem VA-Stahl-Gefäß in 3000 ml 5 M NaOH suspendiert. Die Suspension wurde unter Rühren 30 Mimuten bis auf 90 °C erhitzt. Dabei ging das Polymer vollständig in Lösung. Die noch heiße Lösung wurde anschließend unter Rühren mit 40 Liter Wasser verdünnt. Dabei schied sich das wasserunlösliche Polyhydroxymethylen als amorpher Niederschlag ab. Zur Beschleunigung dieser Abscheidung wurden der Suspension 400 g festes NaCl zugesetzt. Nach drei Stunden wurde die über dem Niederschlag stehende Flüssigkeit größtenteils abgehebert. Der in dem Behälter verbliebene Teil des Ansatzes wurde unter Rühren mit 10 molarer HCl versetzt, bis die Suspension einen pH-Wert von 12 erreicht hatte. Feststoff und Flüssigkeit wurde durch Filtrieren getrennt und der Filterrückstand mit 0,15 molarer NaCl-Lösung von der restlichen Lauge freigewaschen. Das so gewonnene Produkt wurde mit 0,15 molarer NaCl-Lösung zu einer Suspension verrührt, deren pH-Wert mit 1 molarer HCl auf 7,5 eingestellt wurde und auf 3 Liter aufgefüllt.

In dieser Suspension des Polyhydroxymethylenderivates wurden unter Rühren 11 g Trinatriumcitrat gelöst. Mit dieser Suspension wurde anschließend eine Chromatographiesäule mit 19 cm Durchmesser gepackt. 1,4 Liter HumanPlasma, mit Citrationen als Komplexbildner gewonnen, wurden mit 27 µg Hepatitis B-s-Antigen versetzt und über die Säule laufen gelassen.

Die proteinreichsten Fraktionen des Säulendurchflusses wurden vereinigt; sie hatten ein Gesamtvolumen von 1,6 Liter und enthielten 89 % der Proteine des Ausgangsplasmas.

In dem so behandelten Plasma konnte mit den bekannten Methoden weder Cholesterin (CHOD-PAP-Methode) noch ApoB-Protein (turbidimetrische Immunreaktion) oder HBsAg (ELISA-Technik) nachgewiesen werden.

## Patentansprüche

1. Verfahren zum Abtrennen von Lipoproteinen aus einer wässrigen Flüssigkeit, dadurch gekennzeichnet, daß man die Flüssigkeit mit einen Polyhydroxymethylen in Kontakt bringt, das einen aufgepfropften oxethylierten Alkohol oder eine aufgepfropfte oxethylierte Carbonsäure enthält, die Flüssigkeit abtrennt und gegebenenfalls die Lipoproteine vom Polyhydroxymethylen eluiert.

2. Verfahren nach Anspruch 1, dadurch gekennzeichnet, daß der Alkohol ein Alkanol mit 4 bis 30 Kohlenstoffatomen oder die Carbonsäure eine aliphatische Carbonsäure mit 4 bis 20 Kohlenstoffatomen ist.

3. Verfahren nach einem der Ansprüche 1 oder 2, dadurch gekennzeichnet, daß die Flüssigkeit Blutplasma oder -Serum ist.

4. Verwendung eines Polyhydroxymethylens, das einen aufgefropften oxethylierten Alkohol oder eine Aufgepfropfte oxethylierte Carbonsäure enthält zur Gewinnung von praktisch lipoproteinfreiem Plasma oder Serum.

## Claims

1. A process for removing lipoproteins from an aqueous fluid, which comprises bringing the fluid into contact with a polyhydroxymethylene onto which has been grafted an oxyethylated alcohol or an oxyethylated carboxylic acid, separating off the fluid and, where appropriate, eluting the lipoproteins from the polyhydroxymethylene.

2. The process as claimed in claim 1, wherein the alcohol is an alkanol having 4 to 30 carbon atoms, or the carboxylic acid is an aliphatic carboxylic acid having 4 to 20 carbon atoms.

3. The process as claimed in claim 1, wherein the fluid is blood plasma or serum.

4. The use of polyhydroxymethylene onto which has been grafted an oxyethylated alcohol or an oxyethylated carboxylic acid for obtaining a plasma or serum which is virtually free of lipoproteins.

## Revendications

1. Procédé de séparation de lipoprotéines d'un liquide aqueux, caractérisé en ce que l'on met le liquide en contact avec un polyhydroxyméthylène qui comporte un alcool oxéthylé greffé ou un acide carboxylique oxéthylé greffé, en ce que l'on sépare le liquide et en ce qu'éventuellement on élue les lipoprotéines du polyhydroxyméthylène.

2. Procédé selon la revendication 1, caractérisé en ce que l'alcool est un alcanol ayant 4 à 30 atomes de carbone ou que l'acide carboxylique est un acide carboxylique aliphatique ayant 4 à 20 atomes de carbone.

3. Procédé selon l'une des revendications 1 ou 2, caractérisé en ce que le liquide est du plasma ou du sérum sanguin.

4. Emploi d'un polyhydroxyméthylène, qui comporte un alcool oxéthylé greffé ou un acide carboxylique oxéthylé greffé pour l'obtention de plasma ou de sérum pratiquement exempts de lipoprotéines.